# FASCICULE DE BREVET EUROPEEN

(11) **EP 1 443 898 B1**
(45) Date de publication et mention de la délivrance du brevet: **14.01.2009**
(21) Numéro de dépôt: 02788055.8
(22) Date de dépôt: 14.11.2002
(51) Int. Cl.: A61K 8/97, A61Q 17/04, A23L 1/30

(54) **PRODUIT CONTENANT UN EXTRAIT D'ALGUE ROUGE DU GENRE PORPHYRA ET SES UTILISATIONS POUR PROTEGER LES CELLULES**
MITTEL ENTHALTEND EIN ROTE ALGEN EXTRAKT DER GATTUNG PORPHYRA UND IHRE VERWENDUNG ZUM SCHUTZ DER ZELLEN
PRODUCT CONTAINING A RED ALGA EXTRACT OF THE GENUS PORPHYRA AND USES THEREOF FOR PROTECTING CELLS

(30) Priorité: 14.11.2001 FR 0114731; 14.11.2002 FR 0214253
(43) Date de publication de la demande: 11.08.2004
(73) Titulaire: Larena, 75007 Paris (FR)
(72) Inventeur: LECLERC, Christian, F-18130 Dun-sur-Auron (FR); PAUL, François, F-31400 Toulouse (FR)
(74) Mandataire: Breese, Pierre
(86) Numéro de dépôt international: PCT/FR2002/003901
(87) Numéro de publication internationale: WO 2003/041679

(56) Documents cités:
- WO-A-00/70968
- FR-A- 2 655 268
- FR-A- 2 803 200
- FR-A- 2 803 201
- PATENT ABSTRACTS OF JAPAN vol. 1996, no. 01 (C & JP 07 242523 A (MIKIMOTO PHARMACEUT CO)
- DATABASE WPI Week 200053 Derwent Publications Ltd., London, GB; AN 2000-568046 XP002208155 & JP 2000 212056 A (SHISEIDO CO)
- DATABASE WPI Week 199442 Derwent Publications Ltd., London, GB; AN 1994-338190 XP002239697 & JP 06 263623 A (SARIENSU KK)
- DATABASE WPI Week 200256 Derwent Publications Ltd., London, GB; AN 2002-522564 XP002239698 & JP 2002 069443 A (MICRO ALGE CORP KK)

## Description

La présente invention concerne le domaine de la préparation de produits à base d'extraits d'algue qui sont utiles comme compléments alimentaires et dans des applications cosmétiques ou dermatologiques pour la protection cellulaire, en général.

L'algue rouge appartenant au genre *Porphyra* comprend plusieurs espèces dont :
- *Porphyra umbilicalis,* ramassée en Bretagne
- *Porphyra purpurea,* présente en Bretagne
- *Porphyra tenera* cultivée au Japon
- *Porphyra yezoensis* cultivée au Japon
- *Porphyra columbina* ramassée en Australie

Les espèces alimentaires de *Porphyra* sont appelées également Nori. L'espèce *Porphyra umbilicalis* forme une lame fine lancéolée à orbiculaire de couleur violacée qui peut atteindre 60 cm de long. L'espèce Porphyra est l'algue alimentaire la plus consommée dans le monde. En France, l'espèce *Porphyra umbilicalis* est autorisée à la consommation alimentaire humaine. L'intérêt nutritionnel de cette algue réside dans sa richesse en composés divers tels que :
- des protéines, jusqu'à 45 % de la matière sèche,
- des acides gras poly-insaturés, comme l'EPA (acide eicosapentaénoique), jusqu'à 5 % des lipides de l'algue,
- des vitamines du groupe B, la vitamine B12 en particulier,
- de la vitamine A et des caroténoides.

On a décrit dans l'art antérieur de nombreuses propriétés d'extraits de l'algue *Porphyra* et les composés qu'ils contiennent, comme par exemple :
- des propriétés anti-oxydantes d'un extrait méthanolique (80 %) de *Porphyra tenera,*
- des propriétés anti-oxydantes d'extraits dans divers solvants polaires et apolaires de *Porphyra yezoensis,* en particulier de l'extrait méthanolique,
- des propriétés anti-oxydantes d'un extrait réalisé avec du méthanol (50 %) et de l'acétone,
- la présence de concentrations élevées de taurine et de ses dérivés, soluble en milieu hydroalcoolique, connue pour ses propriétés élicitrices des protéines chaperonnes (HSP),
- la présence de dérivés de sucres solubles en milieu hydroalcoolique comme le floridoside (alpha-D-galactopyranosyl-(1-2)-glycérol) et ses isomères, qui jouent un rôle essentiel pour la régulation osmotique du milieu cellulaire soumis à des stress hydriques importants,
- la présence de porphyrane, polysaccharide de la famille des gars , non extrait car non soluble dans des solutions hydroalcooliques ayant une teneur en alcool supérieure à 70 %, qui présentent des propriétés immunostimulantes et anti-tumorales.

La Demanderesse a maintenant mis en évidence qu'un extrait hydroalcoolique de l'algue rouge *Porphyra umbilicalis* présente la capacité d'induire la synthèse des protéines de stress lors de contraintes physiques diverses sur les cellules.

Cette capacité inattendue confère à ces extraits un intérêt remarquable pour la préparation de compositions actives pour protéger les cellules du stress provoqué par des contraintes physiologiques ou physiques comme un choc thermique ou l'exposition aux rayonnements UV ou encore par des contraintes physiopathologiques notamment des infections ou des inflammations.

En effet, la synthèse des protéines de stress décrite en réponse à des traitements qui accélèrent la dénaturation des protéines comme la chaleur, est un moyen de défense généralisé, développé par la cellule pour faire face à une très grande diversité d'agressions. Les protéines de stress s'associent aux protéines dont la structure tridimensionnelle est altérée du fait d'une agression et participent à leur élimination, à leur renaturation ou alors les protéines de stress s'associent aux protéines dont la structure tridimensionnelle n'est pas encore acquise (protéine en cours de biosynthèse) et participent à leur maturation. Les protéines de stress protègent ainsi les cellules en préservant l'intégrité de leurs composants et donc leur fonctionnalité.

L'induction plus précoce de la synthèse des protéines de stress en réponse à une agression et une synthèse plus importante de ces protéines sont donc des avantages pour les cellules à qui l'on confère un système plus efficace de protection.

En conséquence, l'invention a pour objet un produit capable d'induire la synthèse des protéines de stress lors de contraintes physiques physiologiques ou d'agressions physiopathologiques sur les cellules, caractérisé en ce qu'il comprend ou est constitué d'un extrait hydroalcoolique d'algue rouge, appartenant à l'espèce *Porphyra umbilicalis*.

L'extrait d'algue comprend les composés suivants, dans des proportions exprimées en poids par rapport au poids total de l'extrait lorsqu'ils sont dosées :
- Taurine et dérivés : 1 à 5 %,
- Floridoside et dérivés : 10 à 25 %,
- Caroténoïdes et AGPI (non dosés),
- Vitamines hydrosolubles (non dosées),
- Acides aminés et dérivés (non dosés),
- Pigments divers.

Un produit selon l'invention comprend de 1 à 30 % en poids d'un extrait hydroalcoolique d'algue rouge par rapport au poids total du produit.

L'extrait hydroalcoolique d'algue peut être stabilisé par :
- des maltodextrines pour une utilisation ultérieure sous forme de poudre ; un produit en poudre selon l'invention comprend de 10 à 30 %, de préférence de 15 à 25 % et tout préférentiellement de l'ordre de 20 % en poids d'un extrait hydroalcoolique d'algue rouge par rapport au poids total du produit.
- du glycérol pour une utilisation ultérieure sous forme liquide (hors utilisation pour des compositions cosmétiques). Un produit liquide selon l'invention comprend de 10 à 30 %, de préférence de 15 à 25 % et tout préférentiellement de l'ordre de 20 % en poids d'un extrait hydroalcoolique d'algue rouge par rapport au poids total du produit.
- l'addition d'un conservateur (PHENONIP^{®} 0,5 % p/p , par exemple) et une filtration stérilisante (0,2 µ) pour une utilisation dans des compositions cosmétiques ; un produit liquide selon l'invention destiné à l'utilisation dans des compositions cosmétiques comprend de 1 à 25 % et préférentiellement de 3 à 20 % d'un extrait hydroalcoolique d'algue rouge.

Sous forme de poudre, le produit peut être facilement incorporé dans des formulations sèches de compléments alimentaires et conditionné en gélules, comprimés ou sachets, etc.

Sous forme liquide, le produit est utile pour des préparations cosmétiques ou le conditionnement en capsule molle.

L'invention se rapporte à l'utilisation d'un extrait d'algue rouge ou d'un produit le contenant pour la préparation d'une composition, notamment alimentaire ou cosmétique, destinée induire la synthèse des protéines de stress lors de contraintes physiques diverses sur les cellules. Une composition selon l'invention est tout particulièrement utile pour moduler les protéines de stress HSP60, HSP27 et HSP70.

Les travaux de recherche réalisés dans le cadre de l'invention ont permis de mettre en évidence les capacités de l'extrait ou d'un produit le contenant à protéger les cellules contre un choc thermique, les rayonnements UV ou tout autre choc physique ou thérapeutique.

Ces propriétés confèrent aux compositions selon l'invention un intérêt tant chez l'homme que chez l'animal-L'invention concerne l'utilisation de l'extrait d'algue rouge appartenant à l'espèce *Porphyra umbilicalis* ou un produit le contenant pour la préparation d'une composition, notamment alimentaire ou cosmétique, destinée à protéger les cellules contre les stress physiologiques et les agressions physiopathologiques, en particulier les infections et les inflammations.

L'invention concerne également l'utilisation de l'extrait d'algue rouge appartenant à l'espèce *Porphyra umbilicalis* ou un produit le contenant pour la préparation d'une composition, notamment alimentaire ou cosmétique, destinée à protéger les cellules contre des agressions physiques en particulier un choc thermique ou les rayonnements UV.

L'utilisation selon l'invention concerne également les chocs thermiques chauds et les chocs thermiques froids. Ainsi, les compositions selon l'invention peuvent également être utilisées par des individus soumis à des environnements hostiles comme la mer ou la montagne, et conviennent donc spécialement aux plongeurs et aux skieurs, aux alpinistes et aux sportifs de haut niveau.

En ce qui concerne l'exposition aux rayonnements UV, l'invention ne concerne pas l'extrait d'algue rouge ou le produit le contenant comme filtre mais comme agent de protection vis-à-vis du stress subi par les cellules lors d'une telle exposition.

Les compositions de l'invention destinées à protéger les cellules contre des agressions physiques en particulier un choc thermique ou les rayonnements UV peuvent être administrées par voie orale ou par voie topique.

Des compositions selon l'invention peuvent constituer des compléments alimentaires pour l'homme et l'animal apportant par prise de 10 à 1000 mg de préférence de 25 à 250 mg d'un produit contenant l'extrait d'algue rouge.

Une composition selon l'invention constituant un complément alimentaire peut se présenter en gélules, capsules molles, comprimés ou sachets, sirop, etc.

Les compositions selon l'invention peuvent aussi constituer des compositions cosmétiques, éventuellement dermatologiques, qui sont alors appliquées de manière topique sur la peau. Les compositions de l'invention permettent de protéger la peau contre des agressions physiques et plus particulièrement les chocs thermiques ou les rayonnements UV. Les compositions cosmétiques selon l'invention sont également utiles comme compositions anti-âges pour améliorer les qualités et l'apparence de la peau.

Des compositions cosmétiques selon l'invention peuvent se présenter sous toute forme connue de l'homme du métier dans le domaine de la cosmétologie et de la dermatologie sans autre restriction galénique que l'application sur la peau du visage ou du corps. De façon avantageuse, les compositions selon l'invention se présentent sous la forme d'un gel, d'une crème, d'une émulsion, d'un lait, d'un spray, etc....

Ces compositions peuvent aussi comprendre un ou plusieurs agents de formulation ou additifs d'usage connu et classique dans les compositions cosmétiques et dermatologiques tels que, à titre d'exemple et de façon non limitative, des adoucissants, des colorants, des actifs filmogènes, des tensioactifs, des parfums, des conservateurs, des émulsionnants, des huiles, des glycols, des agents absorbeurs de sébum, des vitamines, etc.... Grâce à ces connaissances en matière de cosmétiques, l'homme du métier saura quels agents de formulation ajouter aux compositions de l'invention et en quelles quantités en fonction des propriétés recherchées.

Des compositions cosmétiques selon l'invention comprennent de 0,1 à 5 % et de préférence de 0,5 à 2 % de produit contenant l'extrait d'algue rouge.

D'autres avantages et caractéristiques de l'invention apparaîtront des exemples qui suivent concernant l'activité d'un extrait hydroalcoolique de *Porphyra umbilicalis* sur la réponse au choc thermique ou au rayonnement UV de cellules en culture *in vitro.* Il sera fait référence dans ces exemples aux dessins en annexe dans lesquels :
- La figure 1 représente la cinétique de production de l'hormone de croissance (hGH) par les cellules CHO.
- La figure 2 représente l'analyse par la technique du Western blot du taux des différentes HSP produites par les cellules HeLa après un choc thermique. L'extrait de *Porphyra* est noté E et le témoin T.
- La figure 3 représente l'analyse par la technique du Western blot du taux des différentes HSP produites par les cellules HaCat après irradiation UV. L'extrait de *Porphyra* est noté E et le témoin T.

### Exemple 1 : Activité de l'extrait hydroalcoolique de Porphyra umbilicalis sur la réponse au choc thermique et au rayonnement UV de cellules en culture in vitro

Pour réaliser les expérimentations décrites dans cet exemple, on utilise un extrait hydroalcoolique de *Porphyra* (7 % d'alcool) contenant 0.12 % de matière sèche extraite de l'algue. Cet extrait est ajouté à hauteur de 1 % dans les milieux de culture. La concentration finale en extrait d'algue est de 0.0012 %. Une solution alcoolique de même concentration (7% d'alcool v/v) a été utilisée comme contrôle et ajoutée à hauteur de 1% dans les milieux de culture (concentration finale : 0.07%).

Expérience 1 : mise en évidence de l'induction de la synthèse des HSP par l'extrait de *Porphyra.*

L'extrait de *Porphyra* et la solution contrôle ainsi préparées ont été ajoutées au milieu de culture des cellules CHO (Chinese Hamster Ovary) génétiquement manipulées (technologie TSPc®). Les cellules ont été ensuite soumises à un choc thermique de 43°C pendant 90 min. Dans ce modèle, la synthèse d'hormone de croissance dosée par un kit Elisa (Boehringer-Roche) reflète l'induction de la synthèse d'HSP en réponse à l'agression thermique.

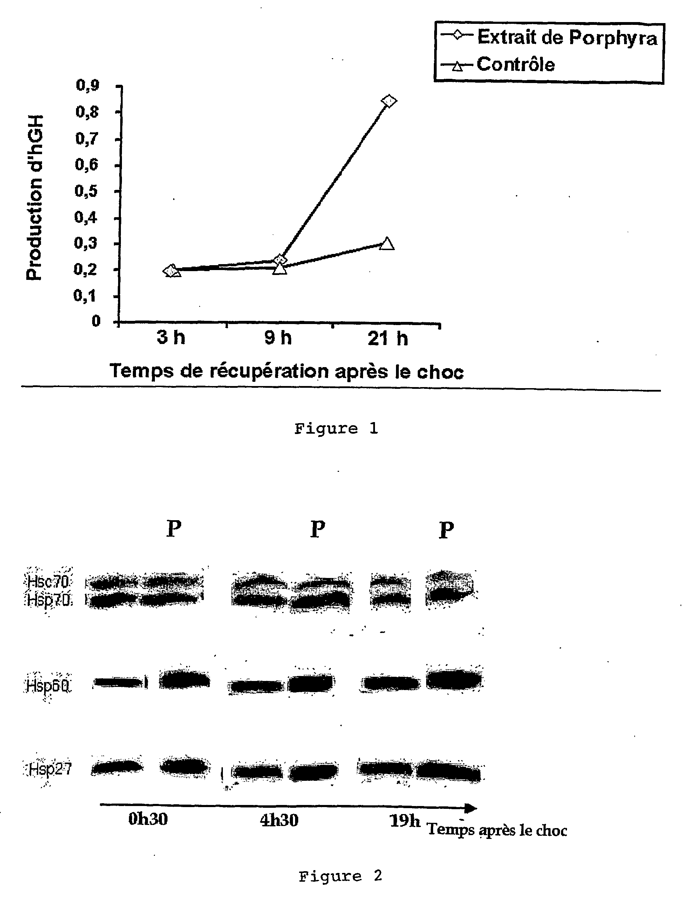

La figure 1 montre que lors du stress thermique, la préincubation des cellules avec l'extrait de *Porphyra*, induit une stimulation de la synthèse d'hGH, donc d'HSP, significativement supérieure à celle observée dans les cellules n'ayant pas reçu l'extrait de *Porphyra.*

Expérience 2 _{:} identification des HSP dont la synthèse est induite par l'extrait de *Porphyra* (choc thermique et UV).

Des anticorps spécifiques anti-HSP27, anti-HSP60, anti-HSP70, et la technique de Western Blot ont été utilisés. Les cellules utilisées sont des cellules HeLa pour le choc thermique et des cellules HaCat pour le stress par les UV (0,250 J/cm² de rayonnement UV à une longueur d'onde de 254 nm) .

La figure 2 montre que l'extrait de *Porphyra* ne modifie pas la production d'HSP en dehors de toute agression, ce qui signifie que l'extrait de *Porphyra* ne modifie pas le mode physiologique de synthèse des HSP. En revanche, lors d'un choc thermique, l'extrait de *Porphyra* stimule la production de protéines HSP60, HSP70 et HSP27. A t = 30 min, on remarque que la synthèse des HSP 27 et 60 est nettement augmentée dans les cellules préincubées avec l'extrait de *Porphyra* par rapport au témoin. En revanche, la synthèse de HSP 70 est équivalente à celle des cellules témoins. Aux temps t = 4h30 et t = 19h après le choc thermique, la synthèse des HSP 27,60 et 70 est significativement plus importante dans les cellules préincubées avec l'extrait de *Porphyra* comparativement aux cellules témoins.

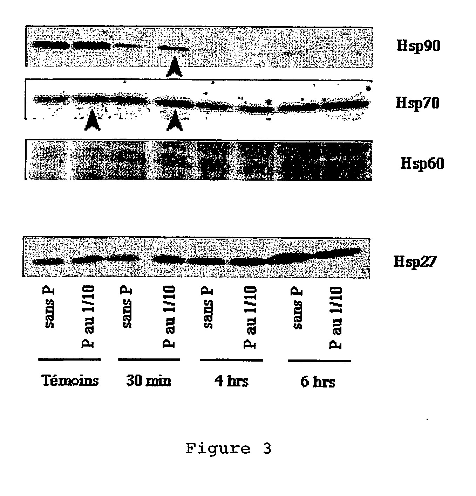

La figure 3 montre que l'extrait de *Porphyra* ne modifie pas la production d'HSP en dehors de toute agression, mais stimule la synthèse par les cellules HaCat des HSP 70 lors d'une irradiation par les UV.

Exemple 2 _{:} Formulations de l'extrait hydroalcoolique de *Porphyra*
1) Complément alimentaire pour l'homme.
30 capsules de 700 mg par étui. Poids net : 21 g. (valeurs arrondies).
Ingrédients : Extrait de *Porphyra* selon l'invention dans le glycérol, huile vierge de bourrache. Capsule (gélatine de poisson et amidon de pomme de terre). Agent d'enrobage : cire jaune d'abeille.
Des vitamines, minéraux, des extraits végétaux et des ferments lactiques peuvent être ajoutés.
2) Complément alimentaire pour l'animal.
30 capsules de 700 mg par étui. Poids net : 21 g. (valeurs arrondies).
Ingrédients : Extrait de *Porphyra* selon l'invention dans le glycérol, huile vierge de bourrache. Capsule (gélatine de poisson et amidon de pomme de terre). Agent d'enrobage : cire jaune d'abeille.
Des vitamines, minéraux, des extraits végétaux et des ferments lactiques peuvent être ajoutés.
3) Composition orale solaire.
45 capsules de 600 mg (TAILLE 6) par étui. Poids net : 27 g. Ingrédients : Capsule (gélatine de polisson et amidon de pomme de terre), huile vierge de bourrache. Extrait de *Porphyra* selon l'invention dans le glycérol, agent d'enrobage : cire d'abeille, extrait de tomate, levure cultivée sur milieu enrichi en sélénium, vitamine E.
4) Composition orale anti-âge pour la peau.
60 capsules de 673 mg par étui. Poids net: 40,4 g. (valeurs arrondies). Ingrédients : huile vierge de bourrache, capsule (gélatine de poisson et amidon de pomme de terre). Extrait sec de thé vert. Agent d'enrobage : cire d'abeille, extrait de *Porphyra* selon l'invention dans le glycérol, hydrolysat de protéines de blé, levure séléniée, sulfate de zinc, sulfate de manganèse.
5) Composition cosmétique anti-âge : soin antirides aux céramides

| **Ingrédients** | **Principes actifs** | **Quantité** | **Unité** | |
|---|---|---|---|---|
| EAU OSMOSEE CHAUDE | | 0,4661 | KG | |
| P O B METHYL SODE (NIPAGINE) | | 0,0020 | KG | |
| BHT (BUTYL HYDROXY TOLUENE) | | 0,0002 | KG | |
| POB PROPYLE PUR (SOLBROL P) | | 0,0010 | KG | |
| EAU OSMOSEE FROIDE | | 0,0300 | KG | |
| TRIETHANOLAMINE 99 % (TEA) | | 0,0024 | KG | |
| ACETATE DE DL ALPHA VIT E | X (Vitamine E) | 0,0050 | KG | |
| HUILE BOURRACHE (CROPURE BORAGE) | X | 0,0025 | KG | |
| PROTULINE | X (Spiruline) | 0,0100 | KG | |
| CONSERVATEUR PARA W7 | | 0,0030 | KG | |
| PARFUM BASE 87 F14892 | | 0,0015 | KG | |
| SOLUTION CARBOPOL 2% | | 0,1500 | KG | |
| EDETA BD | | 0,0005 | KG | |
| GLYCERINE NATURELLE CODEX | X (Humectant) | 0,0300 | KG | |
| MONOPROPYLENE GLYCOL CODEX | | 0,0300 | KG | |
| CERAMIDE VEGETALE PHYTOGLYCOLIPIDE | X | 0,0008 | KG | |
| EMULGADE SE PF (EMULGADE SE) | | 0,0700 | KG | |
| SAFACID 16-18 CODEX | | 0,0250 | KG | |
| SIPOL C 16 PUR | | 0,0100 | KG | |
| LANOL 14 M | | 0,0050 | KG | |
| HUILE GERME DE BLE | | 0,0250 | KG | |
| EUTANOL G | | 0,0600 | KG | |
| CETIOL V | | 0,0400 | KG | |
| SILICONE FLUIDE DC 200 350 CS | | 0,0050 | KG | |
| UVINUL M 40 (EUSOLEX) | X (Filtres UV) | 0,0020 | KG | |
| ESCALOL 557 | X (Filtres UV) | 0,0130 | KG | |
| EXTRAIT DE PORPHYRA | X | 0,0100 | KG | |

6) Composition cosmétique : voile multilamellaire antipollution

| **Ingrédients** | **Principes actifs** | **Quantité** | **Unité** |
|---|---|---|---|
| EAU OSMOSEE FROIDE | | <0,0200 | KG |
| SORBATE DE POTASSIUM | | 0,0020 | KG |
| POB PROPYLE PUR (SOLBROL P) | | 0,0010 | KG |
| BHT (BUTYL HYDROXY TOLUENE) | | 0,0001 | KG |
| EAU OSMOSEE CHAUDE | | 0,0967 | KG |
| NATROSOL 250 HHR | | 0,0035 | KG |
| OLIGOELEMENT CUIVRE | X | 0,0015 | KG |
| OLIGOELEMENT ZINC | X | 0,0015 | KG |
| CONSERVATEUR PARA W7 | | 0,0030 | KG |
| PARFUM COMPOSITION MARINE F 16184 | | 0,0060 | KG |
| EAU OSMOSEE CHAUDE | | 0,5300 | KG |
| GLYCERINE NATURELLE CODEX | X (humectant) | 0,0301 | KG |
| EDETA BD | | 0,0015 | KG |
| POB METHYL PUR (SOLBROL M) | | 0,0020 | KG |
| TRIETHANOLAMINE 99 % (TEA) | | 0,0005 | KG |
| BIOPHILIC H | | 0,0401 | KG |
| EUTANOL G | | 0,0802 | KG |
| LANOL 1688 (CRODAMOL CAP) | | 0,0701 | KG |
| HUILE AMANDE DOUCE STABILISEE | X | 0,0200 | KG |
| HUILE JOJOBA | X | 0,0100 | KG |
| SILICONE FLUIDE DC 200 350 CS | | 0,0200 | KG |
| SILICONE DC 345 | | 0,0200 | KG |
| GELEOL PASTILLES | | 0,0120 | KG |
| ESCALOL 557 | X (filtres UV) | 0,0130 | KG |
| UVINUL M 40 (EUSOLEX) | X (filtres UV) | 0,0020 | KG |
| ACETATE DE DL ALPHA VIT E | X (Vitamine E) | 0,0030 | KG |
| EXTRAIT DE PORPHYRA | X | 0,0100 | KG |

7) Composition cosmétique : sérum réparateur antirides

| **Ingrédients** | **Principes actifs** | **Unité** | **Quantité** |
|---|---|---|---|
| EAU OSMOSEE FROIDE | | | 0,5489 |
| P O B METHYL SODE (NIPAGINE) | | KG | 0,0020 |
| ACIDE LACTIQUE | | KG | 0,0010 |
| SOLUTION COL ROUGE VIF W 3002 A 1/1000 | | KG | 0,0030 |
| NATROSOL 250 HHR | | KG | 0,0015 |
| GLYCERINE NATURELLE CODEX | X (Humectant) | KG | 0,0297 |
| LUBRAGEL MS | X (Hydratant) | KG | 0,1485 |
| ELASTINE MARINE | X | KG | 0,0198 |
| TRITISOL | X (Protéines blé) | KG | 0,0198 |
| CONSERVATEUR PARA W7 | | KG | 0,0030 |
| HYDRUMINE DE PRELE | X | KG | 0,0030 |
| EAU OSMOSEE FROIDE | | KG | 0,1683 |
| PROTULINE | X (Spiruline) | KG | 0,0198 |
| KOTILEN 0 1 VL (POLYSORBATE 80) | | KG | 0,0099 |
| PARFUM COMPOSITION MARINE F 16184 | | KG | 0,0020 |
| EXTRAIT DE PORPHYRA | X | KG | 0,020 |

## Revendications

1. Produit comprenant ou constitué d'un extrait hydroalcoolique d'algue rouge appartenant à l'espèce *Porphyra umbilicalis,* capable d'induire la synthèse de protéines de stress lors de contraintes physiques, physiologiques ou d'agressions physiopathologiques sur les cellules choisies dans le groupe des chocs thermiques, des rayonnements UV, des infections et des inflammations.

2. Produit selon la revendication 1, **caractérisé en ce que** ledit extrait hydroalcoolique de *Porphyra umbilicalis* comprend les composés suivants :
- Taurine et dérivés : 1 à 5 %
- Floridoside et dérivés : 10 à 25 %
- Caroténoïdes et AGPI,
- Vitamines hydrosolubles,
- Acides aminés et dérivés,
- Pigments divers.

3. Produit selon l'une quelconque des revendications 1 ou 2, **caractérisé en ce que** ledit produit comprend de 1 à 30 % en poids dudit extrait hydroalcoolique de *Porphyra umbilicalis* par rapport au poids total du produit.

4. Produit selon l'une quelconque des revendications 1 à 3, **caractérisé en ce que** ledit produit comprend de 15 à 25 % en poids dudit extrait hydroalcoolique de *Porphyra umbilicalis* par rapport au poids total du produit et des maltodexrines, ledit produit étant en poudre.

5. Produit selon l'une quelconque des revendications 1 à 3, **caractérisé en ce que** ledit produit comprend de 15 à 25 % en poids dudit extrait hydroalcoolique de *Porphyra umbilicalis* par rapport au poids total du produit et du glycérol, ledit produit étant liquide.

6. Produit selon la revendication 3, **caractérisé en ce que** ledit produit comprend un agent conservateur utilisable en cosmétique et est soumis à une filtration stérilisante (0,2µ).

7. Utilisation d'un produit selon l'une quelconque des revendications 1 à 6 pour la préparation d'une composition, alimentaire ou cosmétique, destinée à protéger les cellules contre les agressions physiques choisies dans le groupe des chocs thermiques et des rayonnements UV.

8. Utilisation d'un produit selon l'une quelconque des revendications 1 à 6 pour la préparation d'une composition, alimentaire ou cosmétique, destinée à protéger les cellules contre les stress physiologiques et les agressions physiopathologiques choisis dans le groupe des infections et des inflammations.

9. Utilisation alimentaire selon l'une quelconque des revendications 7 ou 8, **caractérisée en ce que** ladite composition apporte par prise de 10 à 1000 mg, de préférence de 25 à 250 mg dudit produit.

10. Utilisation cosmétique selon l'une quelconque des revendications 7 ou 8, **caractérisée en ce que** ladite composition comprend de 0,1 à 5 %, de préférence de 0,5 à 2 % en poids dudit produit.

## Claims

1. Product comprising or consisting of a hydro-alcoholic extract of red algae belonging to the species *Porphyra umbilicalis,* capable of inducing the synthesis of stress proteins during physical, physiological stresses or physiopathological attacks on cells, chosen from the group consisting of heat shocks, UV rays, infections and inflammations.

2. Product according to claim 1, **characterised in that** said hydro-alcoholic extract of *Porphyra umbilicalis* includes the following compounds:
- Taurine and derivatives thereof: 1 to 5%,
- Floridoside and derivatives thereof: 10 to 25%,
- Carotenoids and PUFAs,
- Water-soluble vitamins,
- Amino acids and derivatives thereof,
- Various pigments.

3. Product according to in any of claims 1 or 2, **characterised in that** said product includes from 1 to 30% by weight of said hydro-alcoholic extract of *Porphyra umbilicalis* in relation to the total weight of the product.

4. Product according to in any of claims 1 to 3, **characterised in that** said product includes from 15 to 25% by weight of said extract of *Porphyra umbilicalis,* in relation to the total weight of the product, and maltodextrins, said product being a powder.

5. Product according to in any of claims 1 to 3, **characterised in that** said product includes from 15 to 25% by weight of said extract of *Porphyra umbilicalis,* in relation to the total weight of the product and glycerol, said product being a liquid.

6. Product according to claim 3, **characterised in that** said product includes a cosmetics-grade preservative and is subjected to sterilizing filtration (0.2 µ).

7. Use of a product as claimed in any of claims 1 to 6 for the preparation of a food or cosmetic composition for protecting cells against physical stresses selected from the group of heat shocks and UV rays.

8. Use of a product according to any of claims 1 to 6 for the preparation of a food or cosmetic composition for protecting cells against physiological stresses and physiopathological attacks selected from the group of infections and inflammations.

9. Food use according to any of claims 7 or 8, **characterised in that**, per 10 to 1000-mg sample, said composition preferably contributes from 25 to 250 mg of said product.

10. Cosmetic use as claimed in any of claims 7 or 8, **characterised in that** said composition preferably includes from 0.1 to 5%, and preferably from 0.5 to 2% by weight of said product.

## Patentansprüche

1. Produkt, das einen wässrig-alkoholischen Extrakt von Rotalgen, die zur Spezies *Porphyra umbilicalis* gehören, umfasst oder aus diesem Extrakt besteht, der die Synthese von Stressproteinen bei physischen oder physiologischen Belastungen oder pathophysiologischen Aggressionen in den Zellen induzieren kann, wobei diese aus der Gruppe von thermischen Schocks, UV-Strahlen, Infektionen und Entzündungen ausgewählt sind.

2. Produkt nach Anspruch 1, **dadurch gekennzeichnet, dass** der besagte wässrig-alkoholische Extrakt von *Porphyra umbilicalis* die folgenden Verbindungen umfasst:
- Taurin und Derivate: 1 bis 5%
- Floridosid und Derivate: 10 bis 25%
- Carotinoide und mehrfach ungesättigte Fettsäuren,
- wasserlösliche Vitamine,
- Aminosäuren und Derivate,
- verschiedene Pigmente.

3. Produkt nach einem der Ansprüche 1 oder 2, **dadurch gekennzeichnet, dass** das besagte Produkt 1 bis 30 Gew.-% des besagten wässrig-alkoholischen Extrakts von *Porphyra umbilicalis,* bezogen auf das Gesamtgewicht des Produkts, umfasst.

4. Produkt nach einem der Ansprüche 1 bis 3, **dadurch gekennzeichnet, dass** das besagte Produkt 15 bis 25 Gew.-% des besagten wässrig-alkoholischen Extrakts von *Porphyra umbilicalis,* bezogen auf das Gesamtgewicht des Produkts, und Maltodextrine umfasst, wobei das besagte Produkt pulverförmig ist.

5. Produkt nach einem der Ansprüche 1 bis 3, **dadurch gekennzeichnet, dass** das besagte Produkt 15 bis 25 Gew.-% des besagten wässrig-alkoholischen Extrakts von *Porphyra umbilicalis,* bezogen auf das Gesamtgewicht des Produkts, und Glycerin umfasst, wobei das besagte Produkt flüssig ist.

6. Produkt nach Anspruch 3, **dadurch gekennzeichnet, dass** das besagte Produkt ein Konservierungsmittel umfasst, das in Kosmetika verwendet werden kann, und einer Sterilfiltration (0,2 µm) unterzogen wird.

7. Verwendung eines Produkts nach einem der Ansprüche 1 bis 6 zur Herstellung einer Nahrungsmittel- oder Kosmetikazusammensetzung, die die Zellen vor physischen Aggressionen schützen soll, die aus der Gruppe von thermischen Schocks und UV-Strahlen ausgewählt sind.

8. Verwendung eines Produkts nach einem der Ansprüche 1 bis 6 zur Herstellung einer Nahrungsmittel- oder Kosmetikazusammensetzung, die die Zellen vor physiologischen Stresszuständen und pathophysiologischen Aggressionen schützen soll, die aus der Gruppe von Infektionen und Entzündungen ausgewählt sind.

9. Verwendung in Nahrungsmitteln nach einem der Ansprüche 7 oder 8, **dadurch gekennzeichnet, dass** die besagte Zusammensetzung pro Einnahme 10 bis 1000 mg, vorzugsweise 25 bis 250 mg des besagten Produkts abgibt.

10. Verwendung in Kosmetika nach einem der Ansprüche 7 oder 8, **dadurch gekennzeichnet, dass** die besagte Zusammensetzung pro Einnahme 0,1 bis 5%, vorzugsweise 0,5 bis 2% des besagten Produkts umfasst.
